# EUROPÄISCHE PATENTANMELDUNG

(11) **EP 1 380 262 A1**
(43) Veröffentlichungstag der Anmeldung: **14.01.2004**
(21) Anmeldenummer: 03013392.0
(22) Anmeldetag: 18.06.2003
(51) Int. Cl.: A61B 6/02, A61N 5/10

(54) **System zur Patientenpositionierung für die Strahlentherapie/Radiochirugie basierend auf einer stereoskopischen Röntenanlage**

(30) Priorität: 12.07.2002 DE 10231630
(71) Anmelder: BrainLAB AG, 85551 Kirchheim/Heimstetten (DE)
(72) Erfinder: Wäckerle, Andreas, 86529 Schrobenhausen (DE); Erbel, Stephan, 81677 München (DE); Schlossbauer, Cornel, 80339 München (DE); Fröhlich, Stephan, 85609 Aschheim (DE)
(74) Vertreter: Schwabe - Sandmair - Marx

(57) **Zusammenfassung**

Die Erfindung betrifft ein Patientenpositionierungssystem für die Radiochirurgie bzw. die Radiotherapie, insbesondere mit Hilfe eines LINACs und basierend auf stereoskopischen Röntgenbildern, ein Kalibrierungssystem, insbesondere für ein Patientenpositionierungssystem, eine Bildaufnehmer- bzw. Detektor-Anordnungsvorrichtung, insbesondere zur Verwendung mit einem Patientenpositionierungssystem, und eine Kollisionsschutzeinrichtung, insbesondere zur Verwendung mit einem Patientenpositionierungssystem oder einer Bildaufnehmer- bzw. Detektor-Anordnungsvorrichtung.

## Beschreibung

Die Erfindung betrifft ein System zur Patientenpositionierung für die Strahlentherapie/Radiochirurgie basierend auf einer stereoskopischen Röntgenanlage. Für eine Verbesserung der Radiochirurgie/Strahlentherapie ist eine akkurate Positionierung der Zielregion erforderlich. Stereoskopisches Röntgen und die Verknüpfung mit DRRs stellen eine Möglichkeit für die exakte Positionierung von Patienten für die Strahlentherapie dar. Dabei gilt es folgende Randparameter zu berücksichtigen:
- die Tisch und Gantry-Rotation des LINACs dürfen nicht eingeschränkt werden bzw. die Röntgenanlage darf unter keinen Umständen mit dem Linearbeschleuniger oder dem Patienten kollidieren;
- die Röntgendetektoren dürfen unter keinen Umständen der "harten" Strahlung des LINACs ausgesetzt werden;
- die Zielregion des LINACs (Gebiet um den Zielpunkt herum) soll in beiden Bildern abgebildet werden. Die Bilder können in Behandlungsposition aufgenommen werden.
- die Genauigkeit ist am höchsten, wenn die Anlage ohne (oder mit möglichst wenigen) mechanisch beweglichen Teilen auskommt;
- die Anlage soll jederzeit ohne zusätzliche Rüstzeit betriebsbereit sein. Damit ist das Aus- bzw. Einklappen von Komponenten unerwünscht;
- der Zugang zum Patienten darf nicht eingeschränkt werden;
- kein Teil soll niedriger als 1,90 m angebracht werden, um ein "Kopf-Anschlagen" zu vermeiden;
- es soll weiche kV (40 - 300 kV) Strahlung für eine optimale Bildqualität verwendet werden;
- der Abstand zwischen Objekt (Patient) und Detektor soll möglichst klein sein.

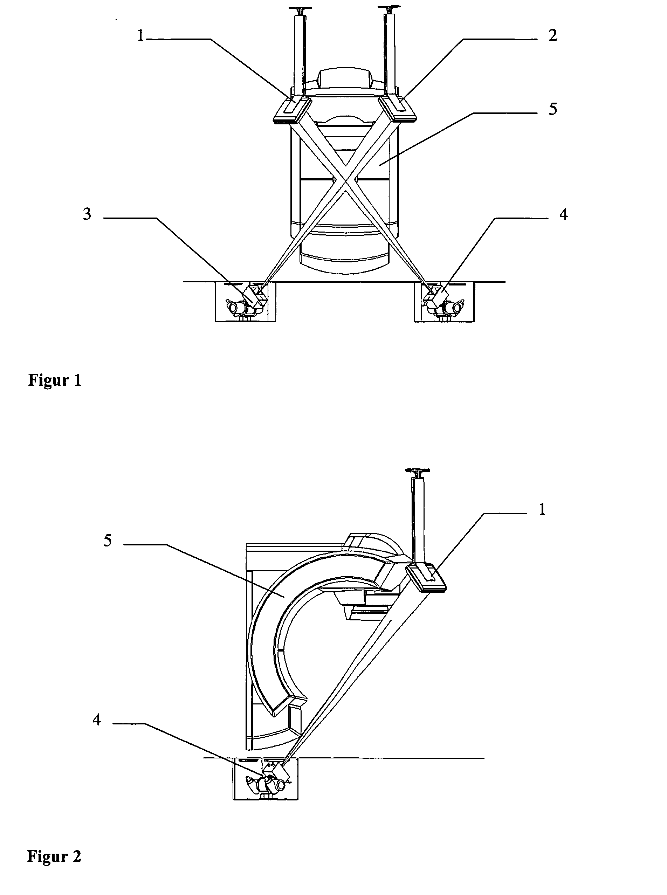

Ein erfindungsgemäßes Patientenpositionierungssystem für die Radiochirurgie bzw. die Radiotherapie, insbesondere mit Hilfe eines LINACs und basierend auf stereoskopischen Röntgenbildern, weist die Merkmale des Anspruchs 1 auf. Die Unteransprüche definieren bevorzugte Ausführungsformen der Erfindung. Vorteilhafterweise weist das System eines oder mehrere der folgenden Merkmale auf:
- die Positionierung beruht auf dem Vergleich von mit dem System geschossenen Röntgenbildern und DRRs;
- zwei kV-Röntgenquellen sind links und rechts der LINAC-Gantry fest im Boden eingelassen. (siehe Figur 1);
- zwei Röntgen-Bildaufnehmer (siehe Figur 1; zum Beispiel a-Si Detektoren oder konventionelle Röntgenbildverstärker) sind links und rechts über dem Patienten, vor der Gantry an der Decke befestigt;
- der Strahlengang von den Röntgenquellen zu den Bildaufnehmern verläuft durch den Zielbereich des Linearbeschleunigers (um das Isozentrum herum);
- jegliche Möglichkeit der Kollision zwischen LINAC und Röntgenanlage ist ausgeschlossen, insbesondere können Gantry und Tisch frei gedreht werden;
- die Bestrahlung soll bis zur Nekrose des Ziels in mehreren Dosen/Bestrahlungsfraktionen, insbesondere in zwei, drei, vier, fünf oder mehr Fraktionen verabreicht werden;
- die Röntgenbilder werden zeitlich beabstandet hintereinander gemacht, insbesondere mit einem Generator für beide Röntgenröhren, der umgeschaltet wird.

Eine Kalibrierung dient zur exakten Bestimmung der Lage (X, Y, Z + Winkel) von Röntgenröhren und Röntgendetektoren im Raum zu einem raumfesten Fixpunkt. Das ist nötig, da der mechanische Aufbau lediglich im Bereich von ein paar Zentimetern genau sein kann, was für die korrekte Funktion des Systems nicht ausreichend ist.

Der "raumfeste Fixpunkt" kann sich an einem beliebigen Punkt im Raum und somit auch im Isozentrum des Linearbeschleunigers befinden.

Wird ein außerhalb des Isozentrums gelegener raumfester Fixpunkt verwendet, so sind grundsätzlich zwei Vorgehensweisen möglich:
a) Das Positionierungssystem wird dazu verwendet, um eine Relation zwischen der Lage der Zielregion und einem oder mehreren "externen Parametern" zu erstellen oder um eine bereits bestehende Relation anzupassen. Unter "externen Parametern" versteht man zum Beispiel die Position von Infrarot-Body-Marker. Die finale Positionierung erfolgt, dann ausschließlich basierend auf den "externen Parametern" und deren Relation zur Zielregion.
b) Der raumfeste Fixpunkt steht in einem definierten Zusammenhang ("Offset") zum Isozentrum. In diesem Fall wird zum ermittelten Positionierungsfehler einfach der Offset addiert.

Um die Elemente der Erfindung möglichst einfach zu erläutern, wird im Folgenden davon ausgegangen, dass der "raumfeste Fixpunkt" sich im Isozentrum des Linearbeschleunigers befindet.

Um die Kalibrierung durchzuführen ist es nötig zu wissen, wo verwendete Röntgenmarker sich relativ zum raumfesten Fixpunkt während der stereoskopischen Röntgenaufnahmen befanden und wohin ihr jeweiliger "Schatten" auf dem Detektor projiziert wird.

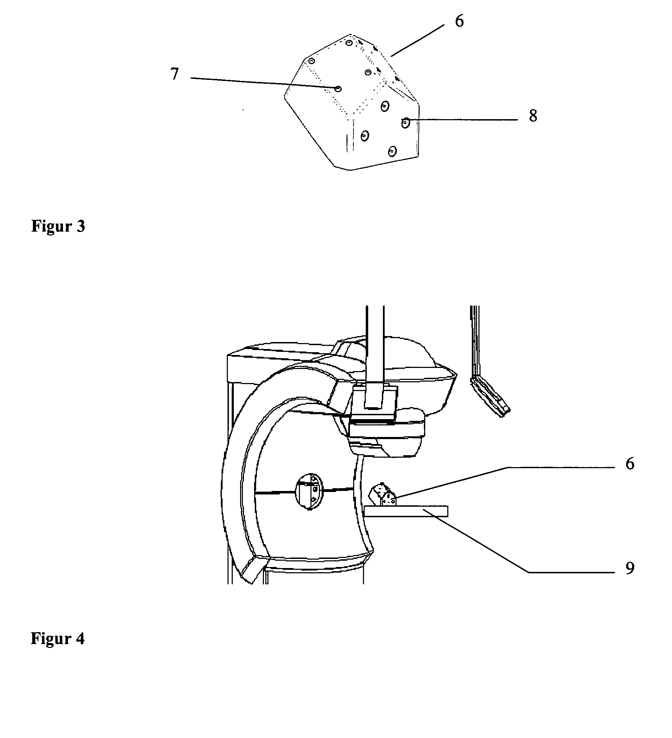

Die Kalibrierung kann im Rahmen der Ausführung der vorliegenden Erfindung unter Verwendung eines oder mehrerer der folgenden Schritte erfolgen:
- zur Kalibrierung wird ein "Phantom" (Figur 3) verwendet, welches mit röntgensichtbaren Markierungen (Röntgenmarker) versehen ist, welche sich im Röntgenbild gut darstellen;
- das besagte Phantom ist zusätzlich mit weiteren Markern bestückt, welche von einem unabhängigen 3D-Messsystem (zum Beispiel IR-Tracking-System) erfasst werden;
- die Röntgenmarker und die zusätzlichen Marker stehen in einem bekannten, räumlich festen Bezug zueinander;
- über die vom 3D-Messsystem in Relation zum raumfesten Fixpunkt erfasste Position der zusätzlichen Marker wird die Raumposition der einzelnen Röntgenmarker errechnet;
- aus der Raumposition der einzelnen Röntgenmarker und deren Projektionen auf die Röntgen-Bildaufnehmer wird die Lage der Röntgenquellen und der Röntgen-Bildaufnehmer im Raum (X, Y, Z + Winkel) relativ zum raumfesten Fixpunkt errechnet;
- die Kalibrierung des Röntgensystems erfolgt unabhängig von der Kalibrierung/Referenzierung des LINAC- Systems;

Die Bildqualität ist besonders vom Abstand Objekt (Patient) -Detektor abhängig. Der Abstand geht quadratisch in die Formel ein. Natürlich gilt das auch für den Abstand Quelle - Objekt; allerdings kann das durch eine höhere Leistung der Quelle besser kompensiert werden.

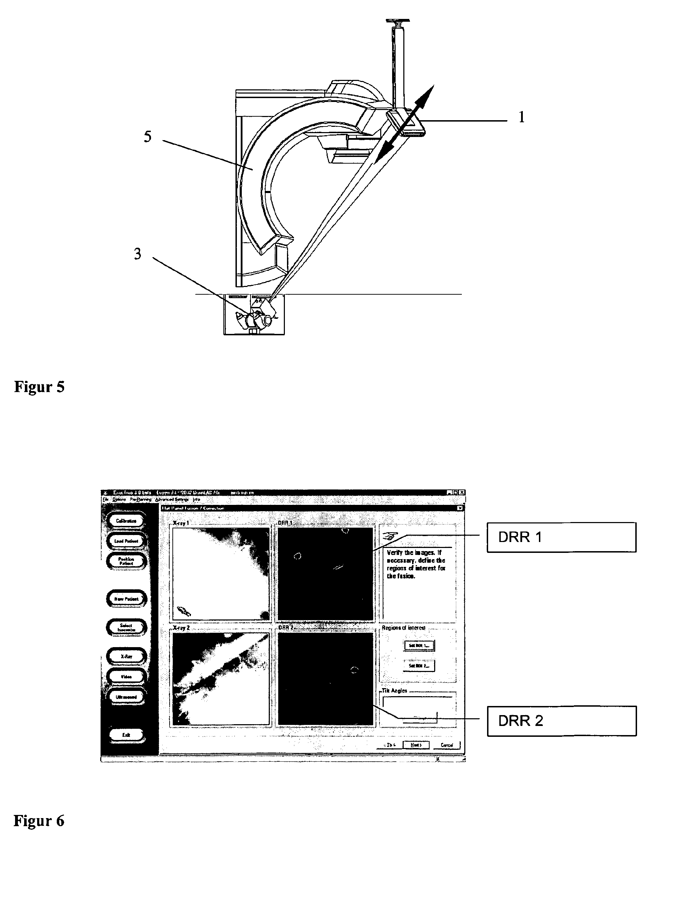

Deshalb ist es vorteilhaft, dass der Detektor möglichst nahe am Patienten sich befindet. Deshalb wird gemäß einem Aspekt der Erfindung ein verfahrbarer Detektor bereitgestellt (Figur 5).

Ein erfindungsgemäßes Patientenpositionierungssystem für die Radiochirurgie bzw. die Radiotherapie, insbesondere mit Hilfe eines LINACs und basierend auf stereoskopischen Röntgenbildern, gemäß dieser Ausführungsform weist eines oder mehrere der folgenden Merkmale auf:
- der besagte Bildaufnehmer (Detektor) kann entlang der Strahlrichtung der jeweiligen Röntgenquelle verfahren werden;
- das Verfahren erfolgt manuell oder motorisch;
- zur Röntgenbildaufnahme befindet der Bildaufnehmer sich in einer patientennahen Position;
- der Bildaufnehmer befindet sich, wenn er nicht benötigt wird, in einer patientenfernen Position;
- die Bildaufnehmer behindern in der patientenfernen Position die Rotation von Gantry und Tisch nicht.

Das erfindungsgemäße System stellt unter anderem einen Kollisionsschutz zur Verfügung, bei dem:
- die Bildaufnehmer mit einem Kollisionsschutzsystem (zum Beispiel Berührungssensor, Ultraschall-Abstandsmesser, Laser Abtastung) ausgerüstet sind und/oder
- das Kollisionsschutzsystem das automatisierte Verfahren der Bildaufnehmer (auch ohne Bediener) ermöglicht.

Alle hierin und in den anhängenden Patentansprüchen beschriebenen Merkmale können einzeln oder in jedweder Kombination als Gegenstand der Erfindung angesehen werden. Die Erfindung wird im Weiteren anhand eines Ausführungsbeispiels und mit Hilfe der beiliegenden Figuren beschrieben, die einzelne Komponenten und Einheiten des erfindungsgemäßen Systems zeigen. Es zeigen:
- Figur 1: ein erfindungsgemäßes System in einer Vorderansicht;
- Figur 2: eine Seitenansicht des Systems;
- Figur 3: ein Kalibrierungs-Phantom;
- Figur 4: ein LINAC-System mit Kalibrierungs-Phantom;
- Figur 5: ein System mit verfahrbarem Bildaufnehmer/Detektor;
- Figur 6: einen Screenshot zur Berechnung der DRRs;
- Figur 7: einen Screenshot zur Überlagerung der DRRs mit den Röntgenbildern;
- Figur 8: einen Screenshot mit Überdeckung der DRRs und der Röntgenbilder; und
- Figur 9: einen Screenshot mit Anzeige der nötigen Korrekturverschiebung.

Das in Figur 1 gezeigte System umfasst zwei links und rechts von der Linearbeschleuniger-Gantry 5 in den Boden eingelassenen konventionellen diagnostischen Röntgenröhren 3, 4 und zwei an der Decke befestigten Röntgendetektoren 1, 2. Es werden in diesen Beispiel Detektoren 1, 2 aus amorphen Silizium verwendet. Der Winkel zwischen den beiden Röhredetektor-Kombinationen beträgt etwas weniger als 90°. Die Figur 2 zeigt eine Seitenansicht des Systems.

Die Kalibrierung dient zur exakten Bestimmung der Lage (X, Y, Z + Winkel) von Röntgenröhren 3, 4 und Röntgendetektoren 1, 2 im Raum relativ zum raumfesten Fixpunkt. Das ist nötig, da der mechanische Aufbau lediglich im Bereich von ein paar Zentimetern genau sein kann, was für die korrekte Funktion des Systems ist dies nicht ausreichend ist.

Zur Kalibrierung wird ein in Figur 3 gezeigtes "Kunststoff-Phantom" 6 verwendet, das sowohl mit Infrarot-Markern 8 versehen ist, und damit vom 3D Tracking System erfasst werden kann, als auch mit Röntgenmarkern 7 (Ringe ähnlich wie Beilagscheiben), welche sich im Röntgenbild gut darstellen. Dieses Phantom 6 wird in der Nähe des raumfesten Fixpunkts positioniert.

Zur eigentlichen Kalibrierung werden zwei Röntgenaufnahmen (je ein Bild pro Röntgenröhre-Röntgendetektor-Kombination) gemacht. Die Lage des Phantoms 6 im Raum wird über ein IR-Trackingsystem (ExacTrac) automatisch erfasst, siehe auch Figur 4. Die Position der Abbildung der Röntgenmarker 7 in den beiden Röntgenbilder wird ebenfalls automatisch detektiert. Mit Hilfe dieser Information kann das System die Lage der Röhren 3, 4 und der Detektoren 1, 2 im Raum errechnen.

### Der Ablauf der Behandlung erfolgt zum Beispiel in der folgenden Weise:

### Vorpositionierung

Der Patient sollte vorteilhafterweise bereits auf ca. plus/minus 3 cm gegenüber dem raumfesten Fixpunkt genau gelagert sein. Die Vorpositionierung kann entweder mit einem Patientenmarker-Trackingsystem oder konventionell auf Basis von Hautmarkierungen, die mit den Raum-Lasern in Deckung gebracht werden, erfolgen. Für die Patientenmarker-Trackingsystem-Vorpositionierung werden Infrarot-Body-Marker verwendet. Das Trackingund Positionierungssystem kann beispielsweise ein ExacTrac-System der Fa. BrainLab, Heimstetten, Deutschland sein. Es wird im weiteren auch so benannt, obwohl andere Tracking- und Positionierungssysteme grundsätzlich auch verwendet werden können.

Die konventionelle Vorpositionierung erfolgt ohne Infrarot-Body-Marker. Es ist anzumerken, dass die nachfolgende Prozedur im Prinzip auch ganz ohne Marker funktionieren würde. Da man aber die Tischbewegung über ExacTrac kontrollieren kann, werden in diesem Fall einige IR-Marker am Tisch vorgesehen.

### Aufnahme der Röntgenbilder

Dann werden zwei Röntgenbilder (je ein Bild pro Röntgenröhre-Röntgendetektor-Kombination) gemacht. Das nötige Umschalten zwischen den beiden Röhren 3, 4 erfolgt manuell. Gemäß einer Ausführungsform macht das System die beiden Röntgenbilder nicht gleichzeitig sondern nacheinander. Dabei besteht die Möglichkeit einen einzigen Generator für beide Röntgenröhren 3, 4 zu verwenden, was Investitionskosten spart. Wegen der Fähigkeit des Systems, den Patienten zu tracken und eine Referenzierung zu jedem Zeitpunkt herzustellen ist der Aufwand, zwei Generatoren bereitzustellen, um gleichzeitige Röntgenbilder zu erstellen, nicht unbedingt notwendig.

Ferner kann, wenn die Bilder nacheinander erstellt werden, sichergestellt werden, dass die Streustrahlung einer Röntgenaufnahme nicht das Bild auf dem anderen Detektor 1, 2 stört. Die Detektoren 1, 2 können dabei vorteilhafterweise sehr nahe an den Zielpunkt und auch sehr nahe aneinander heran gefahren werden, wie zum Beispiel in Figur 5 dargestellt ist; Streufilter können eingespart werden.

### Berechnung der DRRs (Digital Reconstructed Radiographs)

Jetzt werden zwei DRRs berechnet, die den "Soll-Bildinhalt" der Röntgenbilder anzeigen. Ein Beispiel zeigt der Screenshot der Figur 6.

Dazu verwendet das System folgende Informationen: Die Position der Röhren 3, 4 im Raum, die Position der Detektoren 1, 2, die Position des raumfesten Fixpunkts, den 3D CT Datensatz und die Position des geplanten Zielpunkts relativ zum 3D CT-Datensatz (=geplantes Isozentrum) aus dem Planungssystem.

Liegt der Patient bereits absolut richtig, so stimmen die DRRs, von irgendwelchen Helligkeitseinstellungen und Abbildungen der Patientenliege abgesehen, 100 % mit den realen Röntgenbildern überein. Ist die Patientenlagerung nicht korrekt, so weisen die DRR-Röntgenbild-Paare Unterschiede im Bildausschnitt auf.

### Überlagerung und Fusion der DRRs mit den Röntgenbilder

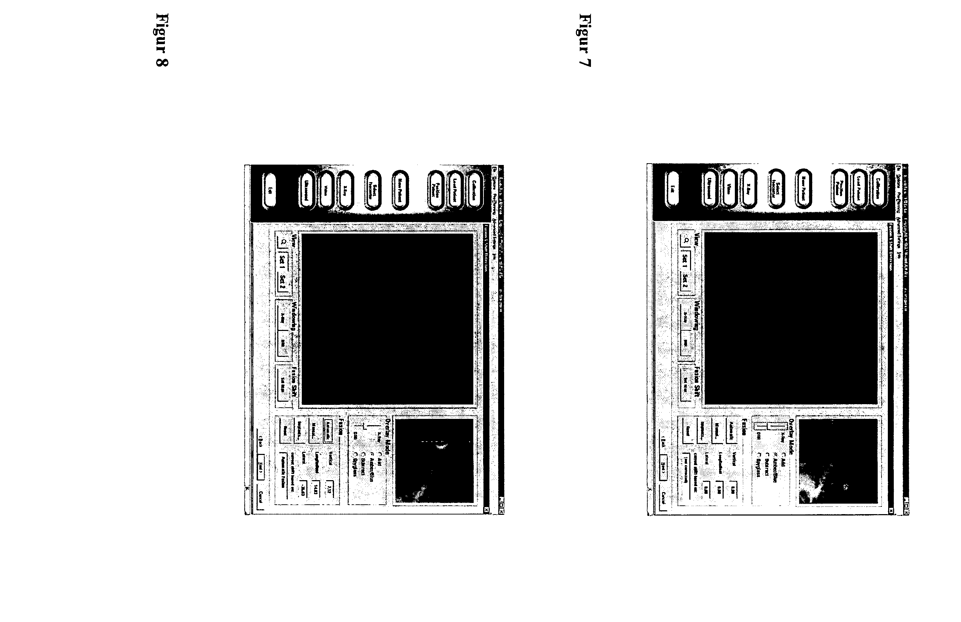

DRRs und Röntgenbilder werden am Bildschirm überlagert, wie beispielsweise im Screenshot der Figur 7 gezeigt ist.

Falls nötig werden dann die Röntgenbilder so verschoben, dass entweder das ganze Bild, ein bestimmter Bildausschnitt oder bestimmte Markierungen in den Bildern (implantierte Patienten-Marker) optimal zur Deckung kommen. Diese Verschiebung kann entweder automatisch durch das System (zum Beispiel Image-Fusion) oder manuell durch den Benutzer erfolgen. Nach der Verschiebung kommen Röntgenbilder und DRRs zur Deckung wie im Screenshot der Figur 8 dargestellt ist. Die 3D-Korrelation der 2D Bilderpaare wird berücksichtigt.

### Berechnung der nötigen Patienten-Verschiebung für eine korrekte Lagerung

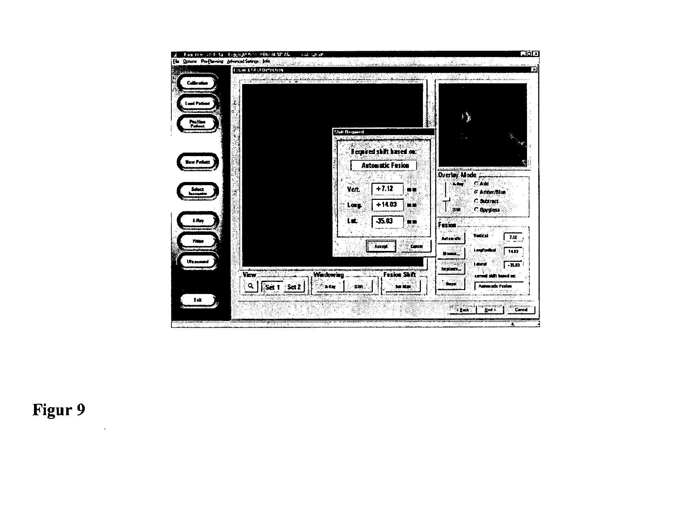

Sofern eine Verschiebung der Röntgenbilder erfolgt ist, wird daraus die eigentliche 3D Fehlpositionierung des Patienten ermittelt und damit wiederum die nötige Korrekturverschiebung des Patienten berechnet. Der Screenshot der Figur 9 zeigt die Anzeige der nötigen Korrekturverschiebung.

### Korrektur des Lagerungsfehlers

Schließlich wird der Patient automatisch (oder manuell aber "computerüberwacht") in die richtige Behandlungs-Position gefahren.

### Verifikation der richtigen Patienten Lagerung (optional)

Prinzipiell kann der Benutzer jetzt manuell einen zweiten Durchlauf des gesamten Ablaufs zu Verifikationszwecken starten. Dabei sollte des System im Rahmen der Systemgenauigkeit (ca. plus/minus 1 mm) keinen weiteren Positionierungsfehler mehr erkennen. Dies kann auch so erfolgen, dass im Verifikationsdurchgang keine Zahlenwerte mehr sondern lediglich die Bildüberlagerung angezeigt wird.

### Patientenüberwachung während der Behandlung

Während der Behandlung überwacht das System die Patientenposition lediglich mit Hilfe der Infrarot-Bodymarker. Eine wiederholte (periodische) Verifikation mit Hilfe des Röntgensystems kann grundsätzlich erfolgen.

## Patentansprüche

1. Patientenpositionierungssystem für die Radiochirurgie bzw. die Radiotherapie, insbesondere mit Hilfe eines LINACs und basierend auf stereoskopischen Röntgenbildern, wobei der Strahlengang für die Erstellung der Röntgenbilder von den Röntgenquellen zu den Bildaufnehmern verläuft durch den Zielbereich des Linearbeschleunigers verläuft, und wobei die Anordnung des LINACs sowie der Röntgenquellen und der Bildaufnahmen so erfolgt, dass die Röntgendetektoren nicht der Strahlung des LINAC ausgesetzt sind.

2. Patientenpositionierungssystem nach Anspruch 1, **gekennzeichnet durch** eines oder mehrere der folgenden Merkmale:
- die Positionierung beruht auf dem Vergleich von mit dem System geschossenen Röntgenbildern und DRRs;
- zwei kV-Röntgenquellen sind links und rechts der LINAC-Gantry fest im Boden eingelassen;
- zwei Röntgen-Bildaufnehmer, zum Beispiel a-Si Detektoren oder konventionelle Röntgenbildverstärker, sind links und rechts über dem Patienten, vor der Gantry an der Decke befestigt;
- jegliche Möglichkeit der Kollision zwischen LINAC und Röntgenanlage ist ausgeschlossen, insbesondere können Gantry und Tisch frei gedreht werden;
- die Bestrahlung soll bis zur Nekrose des Ziels in mehreren Dosen/Bestrahlungsfraktionen, insbesondere in zwei, drei, vier, fünf oder mehr Fraktionen verabreicht werden;
- die Röntgenbilder werden zeitlich beabstandet hintereinander gemacht, insbesondere mit einem Generator für beide Röntgenröhren, der umgeschaltet wird.

3. Kalibrierungssystem für ein Patientenpositionierungssystem gemäß Anspruch 1, **gekennzeichnet durch** eines oder mehrere der folgenden Merkmale:
- zur Kalibrierung wird ein "Phantom" verwendet, welches mit röntgensichtbaren Markierungen versehen ist, welche sich im Röntgenbild gut darstellen;
- das besagte Phantom ist zusätzlich mit weiteren Markern bestückt, welche von einem unabhängigen 3D-Messsystem, zum Beispiel IR-Tracking-System erfasst werden;
- die Röntgenmarker und die zusätzlichen Marker stehen in einem bekannten, räumlich festen Bezug zueinander;
- über die vom 3D-Messsystem in Relation zu einem raumfesten Fixpunkt, insbesondere zum Linearbeschleuniger erfasste Position der zusätzlichen Marker wird die Raumposition der einzelnen Röntgenmarker errechnet;
- aus der Raumposition der einzelnen Röntgenmarker und deren Projektionen auf die Röntgen-Bildaufnehmer wird die Lage der Röntgenquellen und der Röntgen-Bildaufnehmer im Raum (X, Y, Z + Winkel) relativ zu einem raumfesten Fixpunkt, insbesondere zum Linearbeschleuniger errechnet;
- die Kalibrierung des Röntgensystems erfolgt unabhängig von der Kalibrierung/Referenzierung des LINAC- Systems;

4. Bildaufnehmer- bzw. Detektor-Anordnungsvorrichtung zur Verwendung mit einem Patientenpositionierungssystem gemäß Anspruch 1, **gekennzeichnet durch** eines oder mehrere der folgenden Merkmale:
- der Bildaufnehmer kann entlang der Strahlrichtung der jeweiligen Röntgenquelle verfahren werden;
- das Verfahren erfolgt manuell oder motorisch;
- zur Röntgenbildaufnahme befindet der Bildaufnehmer sich in einer dem patientennahen Position;
- der Bildaufnehmer befindet sich, wenn er nicht benötigt wird, in einer patientenfernen Position;
- die Bildaufnehmer behindern in der patientenfernen Position die Rotation von Gantry und Tisch nicht.

5. Kollisionsschutzeinrichtung zur Verwendung mit einem Patientenpositionierungssystem gemäß Anspruch 1 oder einer Bildaufnehmer- bzw. Detektor-Anordnungsvorrichtung gemäß Anspruch 4, **gekennzeichnet durch** eines oder mehrere der folgenden Merkmale:
- die Bildaufnehmer sind mit einem Kollisionsschutzsystem, zum Beispiel Berührungssensor, Ultraschall-Abstandsmesser, Laser Abtastung ausgerüstet;
- das Kollisionsschutzsystem ermöglichst das automatisierte Verfahren der Bildaufnehmer.
